# EUROPEAN PATENT APPLICATION

(11) **EP 0 841 566 A1**
(43) Date of publication of application: **13.05.1998**
(21) Application number: 96810771.4
(22) Date of filing: 11.11.1996
(51) Int. Cl.: G01N 33/96, G01N 33/86, C12Q 1/56, G01N 33/564

(54) **Positive control plasma for lupus anticoagulant**

(71) Applicant: Pentapharm A.G., CH-4052 Basel (CH)
(72) Inventor: Stocker, Kurt, 4147 Aesch (CH); Triplett, Douglas A., Muncie, Indiana 47302 (US); Gempeler-Messina, Patrizia, 4153 Reinach (CH)
(74) Representative: Braun, André, jr.

(57) **Abstract**

It has been found that the addition of positively charged or amphoteric, amphiphilic compounds to normal human plasma simulates the presence of LA and causes a clotting time prolongation in phospholipid-dependent coagulation tests. The prolongation can be corrected by the addition of negatively charged phospholipids. Thus, an artificial control plasma for LA became now available.

## Description

The lupus anticoagulant (LA) is an immunoglobulin (IgG, IgM, or a mixture of both) directed against phospholipids of the blood coagulation system. The presence of LA in plasma interferes with one or more of the in vitro phospholipid dependent tests of coagulation (activated partial thromboplastin time [APTT]; prothrombin time [PT]; dilute Russell's Viper Venom Time [dRVVT]; Textarin Clotting Time [TCT] ; Taipan Venom Time [TVT]) or with the respective chromogenic substrate assays. In all of these phospholipid dependent tests, a prolonged clotting time due to LA will demonstrate either correction or significant shortening upon the addition of excess phospholipid to the test system.

In contrast to specific inhibitors of coagulation proteins, LA has no reactivity with any of the individual coagulation factors. Hence a clotting time prolongation due to LA observed in phospholipid-dependent coagulation tests is not normalized by the addition of normal plasma.

The name 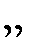 lupus anticoagulant" is a misnomer since the vast majority of patients do not have underlying systemic lupus erythematosus (SLE). More commonly, LA is secondary to infections, drugs (e.g. chlorpromazine, quinidine, procainamide) or it may be seen in an autoimmune disease which has recently been described: Primary Antiphospholipid Antibody Syndrome. LA is also often present in systemic Lupus Erythematosus.

Paradoxically, LA is not associated with clinical bleeding unless there is some associated hemostatic defect (e.g. thrombocytopenia). Approximately 30 to 40% of patients with LA have a history of venous and arterial thromboembolic events. For a number of years, there has been much discussion as to whether LA was causative, a consequence, or coincident with thrombosis. Recent experimental work in animal models suggests that LA and related phospholipid antibodies are a cause of thrombosis. Two recent reviews discuss LA and its closely related antibody: anticardiolipin antibodies [Triplett, D.A: Antiphospholipid - protein antibodies: Laboratory Detection and clinical relevance. Thromb. Res. 78, 1-31 1995; Love P.E., Santoro S.A., Antiphospholipid Antibodies: Anticardiolipin and the Lupus Anticoagulant in Systemic Lupus Erythematosus (SLE) and in non-SLE disorders. Ann. Int. Med. 112, 682-698, 1990].

The laboratory diagnosis of LA is often difficult. Since the APTT is used for screening, there are two major problems. On the one hand commercially available APTT reagents show a wide range of sensitivity and responsiveness to LA. On the other hand, there are a number of clinical situations which are not related to LA, in which an abnormal APTT may be encountered: factor deficiencies, specific inhibitors of factors (e.g. antibodies to factor VIII), the presence of heparin, and acquired multifactor deficiency states such as liver disease and oral anticoagulants. Consequently, it is important for the laboratory to have a systematic sequential approach to the laboratory diagnosis of LA. This requires careful processing of patient plasma and appropriate positive and negative control throughout the investigative evaluation.

The validation of diagnostic procedures for LA assessment would be greatly facilitated if LA containing plasma, quantitatively and qualitatively normal in all clotting factors, were commercially available as a reference material. Due to the lack of a natural positive control plasma, several attempts to prepare artificial LA plasma for this purpose have been made.

The peptide antibiotic polymyxin B showed a variable response to different APTT reagents depending on whether silica, kaolin, celite or ellagic acid was present as a contact activator component [Brandt, J.T. et al. Laboratory identification of lupus anticoagulants: Results of the second international workshop for identification of lupus anticoagulants. Thrombos. Haemostas. 74, 1597-1603, 1995]. Similar problems were seen by the use of the sphyngomyelinase, ARACHnase® (trademark), isolated from the venom of the brown recluse spider *Loxosceles reclusa* [Mc Glasson, D.L. et al. Evaluation of the lupus anticoagulant effects of brown recluse spider venom on normal plasma. Mild Med. Lab. Sci. 18, 95-98, 1989] or with the phospholipase extracted from Cobra (*Naja* species) venom [Kini, R.M., Evans, H.I., Correlation between the enzymatic activity, anticoagulant and antiplatelet effects of phospholipase A2 isoenzymes from *Naja nigricollis* venom, Thrombos. Haemost. 60, 170-173, (1988)].

It was now found that the addition of positively charged or amphoteric, amphiphilic compounds to normal citrated plasma simulates the presence of LA and causes a clotting time prolongation in phospholipid-dependent coagulation tests e.g. APTT, PT, or Textarin test while phospholipid-independent plasma coagulation tests e.g. ecarin clotting time or thrombin clotting time remain unaffected. In further similarity to the effect of LA, it was also found that the addition of negatively charged phospholipid corrects the clotting time prolongation caused by the addition of positively charged or amphoteric amphiphilic compounds to test systems containing phospholipid and calcium ions.

Suitable amphiphilic compounds can be selected from hydrosoluble quaternary ammonium, pyridinium, guanidinium or quinaldinium compounds bearing at least one lipophilic alkyl-, aryl- or aralkyl residue with 8 to 18 C-atoms and, optionally, one sulfonylated or carboxylated alkyl residue. The preferential salt forms of the positively charged amphiphilic compounds are chlorides, bromides and acetates. The compounds to be used as potential plasma additives are with preference selected from commercially available agents currently used as tensides, antiseptics or drugs as exemplified in table 1. A preferential, economically favourable additive which yields an artificial LA+ control plasma showing a positive reaction with a broad variety of LA test systems and a good response to phospholipid supplementation, is cetylpyridinium chloride.

An artificial LA positive control plasma can be prepared by adding the positively charged or amphoteric, amphiphilic compound to a pool of platelet free, normal, human plasma. The additive concentration is selected in order to mimick the plasma clotting time prolongation observed with plasma of LA patients. Dependent on the properties of the additive, the concentration to be added ranges from 0.2 to 0.9 mmoles per liter for positively charged compounds and from 1 to 4 mmoles per liter for amphoteric compounds. Human plasma can be prepared from venous blood, collected and processed according to conventional techniques, under the addition of a suitable anticoagulant e.g. sodium citrate, acid citrate dextrose (ACD) mixture etc. [ Inwood, M.J. and Thomson, S. Hematology and blood bank, In: Lynch's Medical Laboratory Technology (S.S. Raphael, ed.) Vol. 2, pp. 1237 - 1269. W.B. Saunders Co., Philadelphia, London, Toronto, 1976]. A stable control preparation for use after reconstitution with water can be prepared by subdividing the plasma-additive mixture into adequate portions (e.g. vials with 1.00 ml) and freeze drying.

### Example 1. Effect of various additives on plasma APTT

To citrated normal human plasma, obtained from the Swiss Red Cross blood bank, Basel, increasing doses of positively charged and amphoteric, amphiphilic compounds were added and the concentration which approximately doubled the APTT and the Textarin time was calculated from this doses-response study for each additive. No effect of any additive was observed on the ecarin clotting time. The results are shown in table 1.

For the determination of the APTT and the Textarin clotting time, 0.1 ml plasma, 0.1 ml Textarin -phospholipid reagent (18 Textarin units and 50 µg rabbit brain cephalin per ml) or Actin® (APTT reagent, Dade) respectively, and 1 to 10 µl of additive solution were incubated for 3 minutes at 37°C and the clotting time was determined after the addition of 0.1 ml calcium chloride solution, 0.025 M. For the ecarin clotting time determination 0.2 ml plasma and 1 to 10 µl additive solution were incubated for 3 minutes and the clotting time was determined following the addition of 0.1 ml ecarin solution, 16 ecarin units per ml.

**Table 1**

| Requirement of additives for prolongation of plasma APTT and Textarin time | | | |
|---|---|---|---|
| Additive | charge | mmoles additive per liter for clotting time prolongation to | |
| | | 30 sec Textarin clotting time | 60 sec APTT |
| I | + | 0.51 | 0.33 |
| II | + | 0.83 | 0.55 |
| III | + | 0.46 | 0.27 |
| IV | + | 0.86 | 0.51 |
| V | +/- | 1.94 | 3.22 |
| VI | +/- | 1.03 | 1.38 |
| IX | + | 0.43 | 0.30 |
| X | + | 0.40 | 0.28 |
| none (control clotting time) | | 13.2 sec | 28.6 sec |
| I = Tetradecyltrimethylammonium bromide; II = Dodecyltrimethylammonium bromide; III = Hexadecyltrimethylammonium bromide; IV = Dodecyldimethylammonium bromide; V = 3-(Dimethyldodecylammonio)propanesulfonate; VI = N-Hexadecyl-N,N-dimethyl-3-ammonio-1-propanesulfonate; IX = Cetylpyridinium chloride, X = N-Benzyl-N-dodecyl-N-bis-(2 hydroxyethyl)ammonium chloride. | | | |

### Example 2 Preparation of artificial lupus anticoagulant positive control plasma (LA+ control)

250 mg cetylpyridinium chloride dissolved in 5 ml distilled water were added under continuous stirring to 500 ml pooled citrated platelet-poor normal human plasma obtained from the Swiss Red Cross Blood Bank, Basel. The mixture was kept during 1 hr at 4°C and was then subdivided into 1 ml portions, filled into vials and lyophilized. After reconstitution with 1 ml distilled water, this LA+ control plasma showed the properties listed in table 2. Similar as in presence of LA, artificial LA+control plasma showed prolonged clotting times in all the phospholipid-dependent test systems and the clotting time prolongation was significantly reduced after phospholipid supplementation. The phospholipid independent tests remained unaffected in artificial LA+ control plasma.

**Table 2**

| Phospholipid dependent and independent coagulation tests with normal plasma, artificial LA+ control and plasma of a patient with lupus anticoagulant, and effect of phospholipid supplementation. | | | |
|---|---|---|---|
| Clotting time [s] | | | |
| Test | LA+control | normal | LA patient |
| APTT | 83.7 | <36.2 | 56.6 |
| +20 µg PL | 48.0 | | 38.0 |
| RVV | 54.9 | <38.0 | 49.0 |
| +20 µg PL | 27.8 | | 37.0 |
| Textarin | 25.1 | 18-22 | 41.6 |
| +20 µg PL | 19.2 | | 22.0 |
| Thromboplastin time | 174.7 | 11.5-13.0 | 14.7 |
| +20 µg PL | 80.7 | | 11.0 |
| Ecarin | 18.0 | 18-21 | 18.0 |
| Thrombin | 18.6 | <20.0 | 17.0 |
| APTT, ecarin- and Textarin tests were performed as described in example 1. RVV-clotting time was measured in a test mixture composed of 50 µl plasma, 50 µl cephalin, 50 µg/ml, 50 µl RVV-X, 1 µg/ml, and, following 3 minutes pre-incubation at 37°C, 50 µl calcium chloride solution, 25 mM. The thrombin clotting time was determined at 37°C, after the addition of 100 µl thrombin solution, 3 NIH unit/ml to 100 µl plasma. | | | |

### Example 3

Artificial LA+ control plasma prepared according to example 2 was tested in comparison to normal plasma, using different commercial diagnostic reagents for lupus anticoagulant. The tests were performed following the instructions of the manufacturers. As shown in table 3, all tests gave prolonged clotting times with LA+ control as compared with normal plasma.

**Table 3**

| Coagulation times of normal plasma and artificial LA+ control measured with various commercial LA test reagents. | | |
|---|---|---|
| Clotting time [s] | | |
| Test | normal plasma | LA+ plasma |
| APTT, Actin FSL, DADE | 38.8 | 149.4 |
| Actin, DADE | 39.6 | >300 |
| PT(..) | 17.0 | 47.3 |
| dRVVT (American Diagnostica) | 43.6 | 89.3 |
| dRVVT confirm (American Diagnostica) | 40.2 | 71.7 |
| Staclot LA (Diagnostica Stago) | 52.9 | 62.7 |

## Claims

1. Artificial control plasma for LA comprising a positively charged or amphoteric, amphiphilic compound or its salt for prolonging the clotting time in phospholipid-dependent plasma coagulation tests, while the clotting time of phospholipid-independent plasma coagulation tests remains unaffected.

2. Compound or its salt according to claim 1, comprising a hydrosoluble quaternary ammonium, pyridinium, guanidinium or quinaldinium compound bearing at least one lipophilic alkyl-, aryl- or aralkyl residue with 8-18 C-atoms and, optionally, one sulfonylated or carboxylated alkyl residue.

3. Chloride, bromide or acetate salts of the positively charged amphiphilic compound according to claims 1 or 2.

4. Compound or its salt according to one of claims 1-3 comprising one or more tensides, antiseptics or drugs, especially tetradecyltrimethylammonium bromide, dodecyltrimethylammonium bromide, hexadecyltrimethylammonium bromide, dodecyldimethylammonium bromide, 3-(dimethyldodecylammonio)propanesulfonate, N-hexadecyl-N,N-dimethyl-3-ammonio-1-propanesulfonate, N-benzyl-N-dodecyl-N-bis-(2-hydroxyethyl)ammonium chloride, and/or cetylpyridinium chloride.

5. Negatively charged phospholipids to correct the prolongation of the plasma clotting time caused by the compound according to one of claims 1-4.

6. Method for the preparation of an artificial control plasma for LA, characterized by adding the positively charged or amphoteric, amphiphilic compound according to one of claims 1-4 to platelet free human plasma.

7. Measuring of LA in human plasma characterized by comparing the prolongation of the clotting time of the artificial control plasma for LA according to claim 1 and the plasma of the patient with LA with human plasma without LA during phospholipid dependent coagulation tests and optionally correcting the prolongation of the clotting time caused by LA and the artificial control plasma for LA according to claim 1 by the addition of negatively charged phospholipids.
